# EUROPEAN PATENT APPLICATION

(11) **EP 2 362 320 A1**
(43) Date of publication of application: **31.08.2011**
(21) Application number: 11155344.2
(22) Date of filing: 22.02.2011
(51) Int. Cl.: G06F 17/30, G06F 19/00

(54) **Clinical test information managing apparatus and non-transitory storage medium**

(30) Priority: 25.02.2010 JP 2010039847
(71) Applicant: SYSMEX CORPORATION, Kobe-shi, Hyogo 651-0073 (JP)
(72) Inventor: Luscombe, Steve, Hyogo 651-0073 (JP); Kantroo, Manoj, Hyogo 651-0073 (JP); Segrove, Christopher, Hyogo 651-0073 (JP)
(74) Representative: HOFFMANN EITLE

(57) **Abstract**

The present invention is a clinical test information managing apparatus including at least one display configured to display cascading tiers of clinical test information, the apparatus further including at least one processor of a computer system and at least one memory that stores programs executable by the at least one processor to: display a first tier of clinical test information including a listing of items which include process stages that show progresses of testing, associated with an indication of presence of samples which are situated at a respective stage; and when receiving a selection by a user among the items, display a second tier of clinical test information including a listing of identifications of samples which are identified by the indication of presence associated with a selected item.

## Description

### FIELD OF THE INVENTION

The present invention relates to a clinical test information managing apparatus, and a non-transitory storage medium for managing information related to a sample test by a clinical sample analyzer.

### BACKGROUND

A clinical test information managing apparatus called a WAM (Work Area Manager) or a LIS (Laboratory Information System), connected to a plurality of clinical sample analyzers, for receiving test information transmitted from the clinical sample analyzer, and storing and displaying the same is known.

For such clinical test information managing apparatus, Japanese Patent Publication No. H08-233825 describes a data managing apparatus which receives measurement results from a plurality of analyzers, and displays the progress situation of the measurements in the plurality of analyzers in association with corresponding analyzer and sample on one process management screen for every measurement field (e.g., biochemical, blood, blood serum etc.).

However, in the data managing apparatus described in Japanese Patent Publication No. H08-233825, the entire process management screen needs to be looked to check the presence of a sample in a specific process such as waiting for first measurement, waiting for retest, and the like , which is troublesome. In particular, if there are great number of specimens or analyzers, switching of screens, operations such as scrolling, and the like are necessary for many times, which is troublesome.

### SUMMARY

The scope of the present invention is defined solely by the appended claims, and is not affected to any degree by the statements within this summary.

A first aspect of the present invention is a clinical test information managing apparatus comprising at least one display configured to display cascading tiers of clinical test information, the apparatus further comprising at least one processor of a computer system and at least one memory that stores programs executable by the at least one processor to: display a first tier of clinical test information comprising a listing of items which comprise process stages that show progresses of testing, associated with an indication of presence of samples which are situated at a respective stage; and when receiving a selection by a user among the items, display a second tier of clinical test information comprising a listing of identifications of samples which are identified by the indication of presence associated with a selected item.

According to the above configuration, a listing of items which comprise process stages is displayed in the first tier of information associated with the indication of presence of samples which are situated at a respective stage. Therefore, the user can easily grasp the presence of the sample in the respective stage by simply checking the first tier of clinical test.

Preferably, the indication of presence is a number of samples situated at the respective stage.

Preferably, the items in the first tier of clinical test information further comprise a type of an error, associated with an indication of presence of samples which sustain the error.

Preferably, the second tier of clinical test information further comprises names of patients from whom the samples come.

Preferably, when receiving a selection by a user among the identifications of the samples in the second tier of clinical test information, the at least one processor displays a third tier of clinical test information comprising patient information of a selected sample and any test results of the selected sample.

Preferably, the at least one processor displays the items of the first tier of clinical test information by way of icons representing the items.

Preferably, the at least one processor updates the clinical test information either automatically on a regular basis or upon receiving an instruction by a user.

Preferably, the type of error indicates that a sample has no identification or an invalid identification.

Preferably, the type of error indicates that a test order is missing for a sample.

Preferably, the process stages comprise: a stage that indicates that a sample is waiting for initial testing; a stage that indicates that initial testing for a sample is in progress; and a stage that indicates that a test results for a sample is waiting for acknowledgement.

Preferably, the process stages comprise: a stage that indicates that a sample is waiting for re-testing; a stage that indicates that re-testing is in progress on a sample;

Preferably, when receiving a selection of the stage in the first tier of clinical test information which indicates that a sample is waiting for initial testing, the at least one processor displays the second tier of clinical test information which comprises a time at which a test order is received for a respective sample.

Preferably, when receiving a selection of the stage in the first tier of clinical test information which indicates that a sample is waiting for re-testing, the at least one processor displays the second tier of clinical test information which comprises a type of re-test to be performed on a sample and a position of a sample if the sample is with other samples in a rack.

Preferably, when receiving a selection of the stage in the first tier of clinical test information which indicates that a test results for a sample is waiting for acknowledgement, the at least one processor displays the second tier of clinical information which comprises a listing of identifications of samples which are situated in the selected stage, and when further receiving a selection of an sample identification in the second tier of clinical test information, the at least one processor displays the third tier of clinical test information which comprises test results of the sample, which may be acknowledged by a user on display.

A second aspect of the present invention is a non-transitory storage medium which stores computer-executable programs executed by at least one processor of a computer system to: display a first tier of clinical test information comprising a listing of items which comprise process stages that show progresses of testing, associated with an indication of presence of samples which are situated at a respective stage; and when receiving a selection by a user among the items, display a second tier of clinical test information comprising a listing of identifications of samples which are identified by the indication of presence associated with a selected item.

### BRIEF DESCRIPTION OF THE DRAWINGS

Fig. 1 is a view showing a hospital clinical test apparatus group including a clinical test information managing apparatus according to the embodiment;
Fig. 2 is a schematic view showing a partial configuration of the clinical test information managing system according to the embodiment;
Fig. 3 is a block diagram showing a schematic configuration of a measurement unit arranged in a blood cell analyzer according to the embodiment;
Fig. 4 is a block diagram showing a configuration of the information processing unit arranged in the blood cell analyzer according to the embodiment;
Fig. 5 is a block diagram showing the configuration of the clinical test information managing server according to the embodiment;
Fig. 6 is a schematic view showing a structure of a process management table;
Fig. 7 is a block diagram showing a configuration of a client device according to the embodiment;
Fig. 8 is a flowchart showing a flow of the entire clinical test;
Fig. 9 is a view showing the transition of the clinical test process in the present embodiment, that is, the transition of the process information registered in F5 of the process management table;
Fig. 10 is a flowchart showing a flow of a measurement order registration process of the clinical test information managing server according to the embodiment;
Fig. 11 is a flowchart showing a flow of a measurement order inquiry accepting process of the clinical test information managing server according to the embodiment;
Fig. 12 is a flowchart showing a flow of a test result accepting process of the clinical test information managing server according to the embodiment;
Fig. 13 is a flowchart showing a flow of an error notifying information accepting process of the clinical test information managing server according to the embodiment;
Fig. 14 is a flowchart showing a flow of a display process of the clinical test information management screen of the clinical test information managing server according to the embodiment;
Fig. 15 is a view showing one example of a menu screen;
Fig. 16 is a view showing one example of a measurement awaiting sample list screen;
Fig. 17 is a view showing one example of a remeasurement awaiting sample list screen;
Fig. 18 is a view showing one example of an acknowledgement awaiting sample list screen;
Fig. 19 is a view showing one part of a detailed information screen; and
Fig. 20 is a view showing a variant of the menu screen.

### DETAILED DESCRIPTION OF THE EMBODIMENT

The preferred embodiments of the present invention will be described in detail below with reference to the drawings.

### [Configuration of clinical test information managing system]

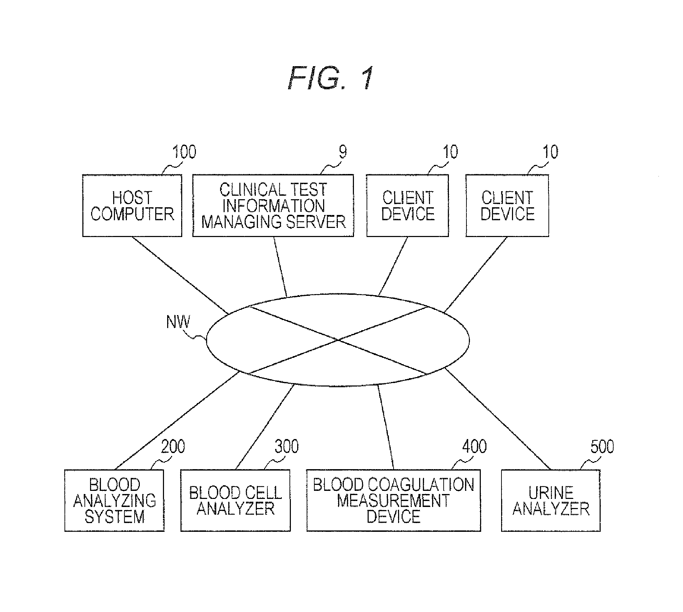

Fig. 1 is a view showing a hospital clinical test apparatus group including a clinical test information managing apparatus according to the present embodiment. In hospitals, a host computer 100, a clinical test information managing server 9 or a clinical test information managing apparatus, a client device 10, a blood analyzing system 200, a blood cell analyzer 300, a blood coagulation measurement device 400, and a urine analyzer 500 are connected to each other through a communication network NW configured by a LAN (Local Area Network). The host computer 100 is a server at the higher level of the clinical test information managing server 9 and manages the operations of the entire test department in the hospital. The host computer 100 also transmits a measurement order to the clinical test information managing server 9. The clinical test information managing server 9 manages measurement progress, measurement result, and the like by the clinical sample analyzer belonging to a specific test field. In the present embodiment, the clinical test information managing server 9 manages the measurement progress, the measurement result, and the like by the blood analyzing system 200, the blood cell counting device 300. the blood coagulation measurement device 400, and the urine analyzer 500. The client device 10 is a computer used to input data to the clinical test information managing server 9 and to browse through a display screen. In the present example, the clinical test information managing server 9, two client devices 10. the blood analyzing system 200, the blood cell counting device 300, the blood coagulation measurement device 400, and the urine analyzer 500 configure the clinical test information managing system.

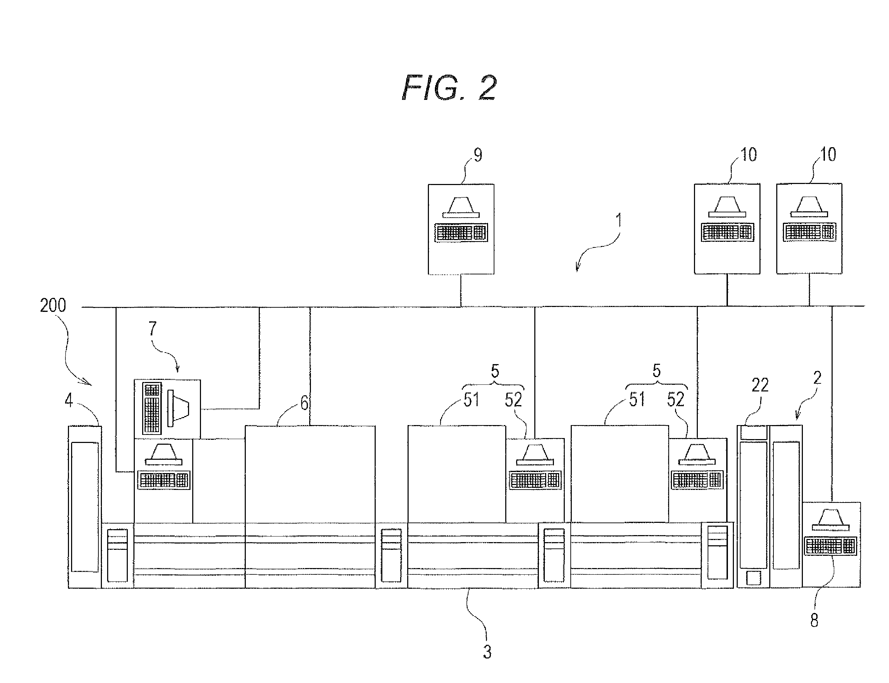

Fig. 2 is a schematic view showing a partial configuration of the clinical test information managing system according to the present embodiment. In other words, Fig. 2 shows the configuration of only the clinical test information managing server 9, two client devices 10, and the blood analyzing system 200. To simplify the description, only the clinical test information managing server 9, two client devices 10, and the blood analyzing system 200 will be described below.

### <Configuration of blood analyzing system 200>

The blood analyzing system 200 is mainly configured by a sample inserting device 2, a sample transporting device 3, a sample accommodating device 4, a blood cell analyzer 5 or a clinical sample analyzer, a smear creating device 6, a blood cell image display device 7, and a system control device 8. The sample inserting device 2 includes a barcode reading unit 22, and reads a sample ID from a plurality of sample containers held in a sample rack, and transports the sample rack, which reading of the sample ID is finished, to the sample transporting device 3. The sample transporting device 3 selectively transports the sample rack to the blood cell analyzer 5, the smear creating device 6, and the blood cell image display device 7. The sample accommodating device 4 receives and accommodates the sample rack, which transportation by the sample transporting device 3 is completed. The blood smear creating device automatically creates a blood smear on a slide glass. The blood cell image display device 7 images a blood smear, automatically analyzes the obtained specimen image, and classifies the sub-class of white blood cells.

### configuration of blood cell analyzer 5>

The blood cell analyzer 5 is a multi-item blood cell analyzer of an optical flow cytometry method that acquires a side scattered light intensity, a fluorescent intensity, and the like regarding the blood cells contained in the blood sample, classifies the blood cells contained in the sample based thereon, counts the number of blood cells for each type, creates a scattergram in which the classified blood cells are distinguished by color for each type, and displays the same. The blood cell analyzer 5 includes a measurement unit 51 for measuring the blood sample and an information processing unit 52 for processing the measurement data output from the measurement unit 51 and displaying the analysis result of the blood sample.

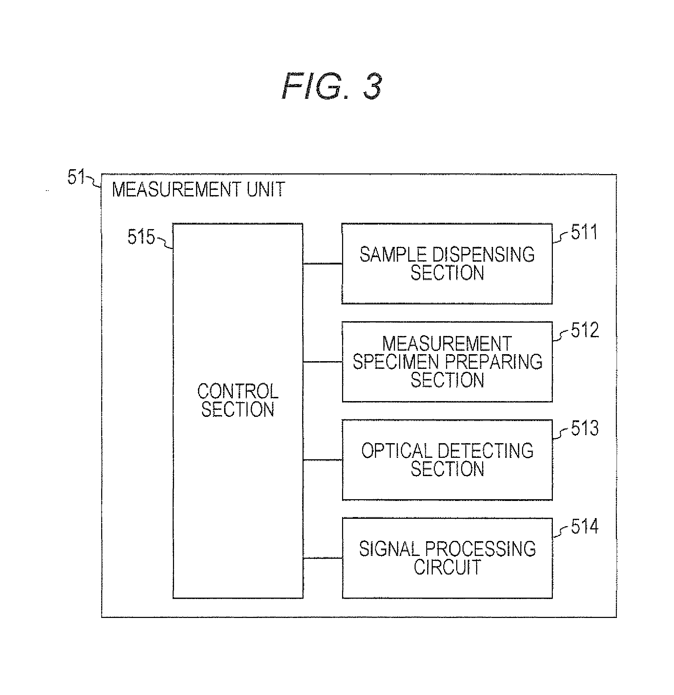

Fig. 3 is a block diagram showing a schematic configuration of the measurement unit 51. The measurement unit 51 includes a samples dispensing section 511, a measurement specimen preparing section 512, an optical detecting section 513, a signal processing circuit 514, and a control section 515.

The sample dispensing section 511 includes a suction tube (not shown), and pierces the suction tube through the lid of the sample container transported on the measurement line of the sample transporting device 3 to suck the blood sample from the sample container. The measurement specimen preparing section 512 includes a mixing container (not shown), and mixes and stirs the blood sample dispensed by the sample dispensing section 511, a reagent, and a diluting solution to prepare a measurement specimen.

The optical detecting section 513 includes a flow cell (not shown), and forms a thin flow of the measurement specimen by supplying the measurement specimen to the flow cell, irradiates the measurement specimen with light, and acquires a side scattered optical signal, a forward scattered optical signal, and a fluorescent signal from an optical sensor. Such signals are output to the signal processing circuit 514. The signal processing circuit 514 is a circuit for processing the electric signals output from the optical detecting section 513. Such signal processing circuit 514 acquires parameters such as the peak and the pulse width of the side scattered optical signal, the forward scattered optical signal, and the fluorescent signal.

The control section 515 includes a CPU and a memory, and is data communicably connected to the sample transporting device 3. The control section 515 controls the sample dispensing section 511, the measurement specimen preparing section 512, the optical detecting section 513, and the signal processing circuit 514 according to the analyzed items provided from the sample transporting device 3, and executes the measurement operations corresponding to the analyzed items. The control section is also configured to transmit the measurement data including the parameters obtained by the signal processing circuit 514 to the information processing unit 52.

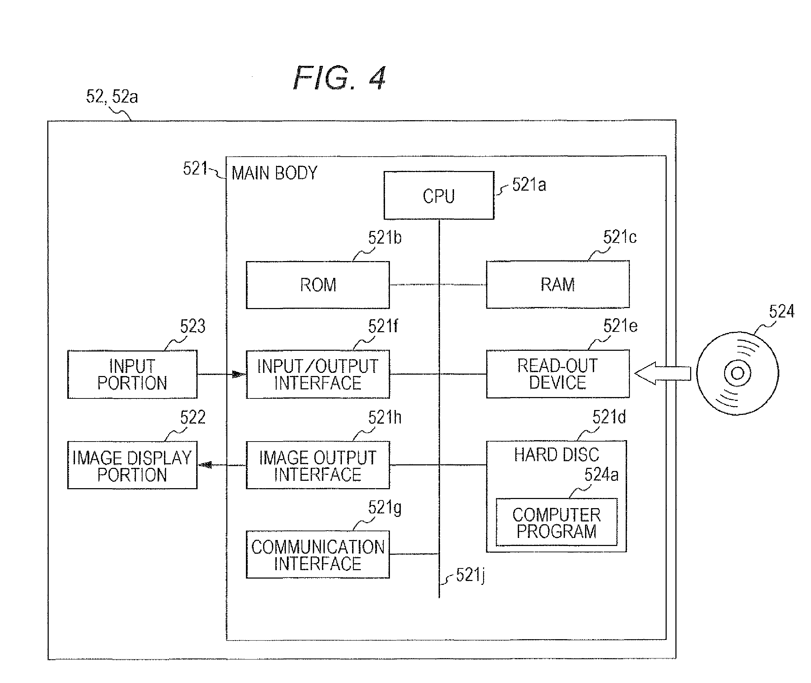

The configuration of the information processing unit 52 will now be described. The information processing unit 52 is configured by a computer. Fig. 4 is a block diagram showing a configuration of the information processing unit 52. The information processing unit 52 is configured by a computer 52a. As shown in Fig. 4, the computer 52a includes a main body 521, an image display portion 522, and an input portion 523. The main body 521 includes a CPU 521 a, a ROM 521b, a RAM 521c, a hard disc 521d, a read-out device 521e, an input/output interface 521f a communication interface 521g, and an image output interface 521h, where the CPU 521a, the ROM 521b, the RAM 521c, the hard disc 521d, the read-out device 521e, the input/output interface 521f, the communication interface 521g, and the image output interface 521h are connected by a bus 521j.

The read-out device 521 e can read out a computer program 524a for causing the computer to function as the information processing unit 52 from a portable recording medium 524, and install the computer program 524a in the hard disc 521d.

### <Configuration of system control device 8>

The system control device 8 is realized by a computer including a CPU, a ROM, a RAM, a hard disc, and the like. The computer functions as the system control device 8 when the CPU executes a system control program installed in the hard disc. The system control device 8 receives the sample ID from the sample inserting device 2, and acquires the measurement order from the clinical test information managing server 9 with the sample ID as the key. The system control device 8 also transmits the measurement order to the samples transporting device 3.

### <Configuration of clinical test information managing server 9>

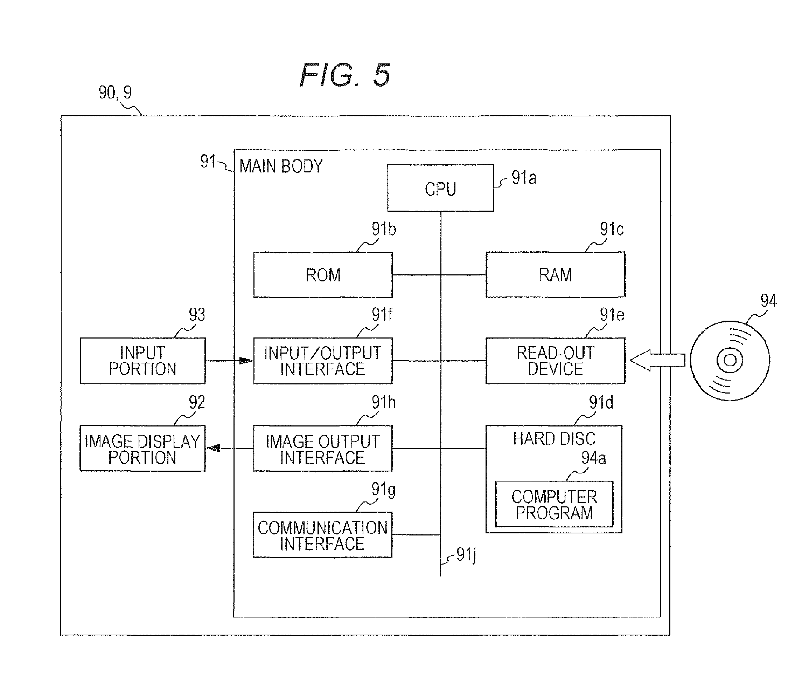

The configuration of the clinical test information managing server 9 will now be described. Fig. 5 is a block diagram showing the configuration of the clinical test information managing server 9 according to the present embodiment. The clinical test information managing server 9 is realized by a computer 90. As shown in Fig. 5, the computer 90 includes a main body 91, an image display portion 92, and an input portion 93 . The main body 91 includes a CPU 91a, a ROM 91b, a RAM 91c, a hard disc 91d, a read-out device 91e, an input/output interface 91f, communication interfaces 91 g, 91h, 91i, and an image output interface 91j, where the CPU 91 a, the ROM 91b, the RAM 91c, the hard disc 91d, the read-out device 91 e, the input/output interface 91f, the communication interface 9 1 g, the communication interface 9 1 h, the communication interface 91i, and the image output interface 91j are connected by a bus 91k.

The read-out device 91e can read out a computer program 94a for causing the computer to function as the clinical test information managing server 9 from a portable recording medium 94, and install the computer program 94a in the hard disc 91d.

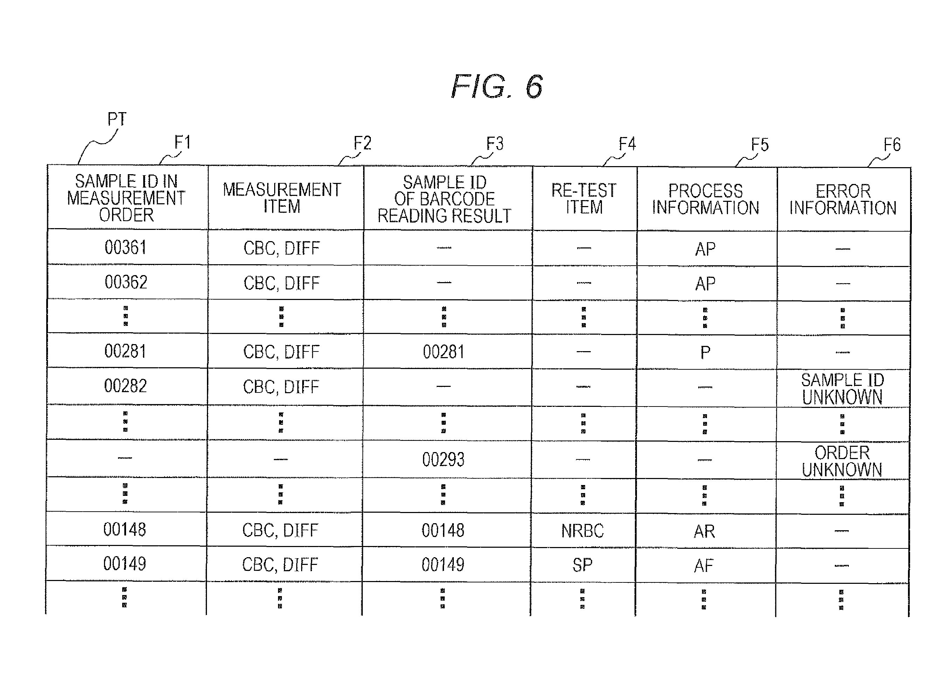

The hard disc 91d includes a region for a process management table PT for managing the progress situation of the clinical test for each sample. Fig. 6 is a schematic view showing a structure of a process management table PT. The clinical test information server 9 communicates with the host computer 100, and receives the measurement order from the host computer 100. The received measurement order includes a sample ID for identifying the sample and a measurement item for the relevant sample. The received measurement order is stored (registered) in the process management table PT. The clinical test information server 9 receives a transmission request of the measurement order from the system control device 8, and transmits the requested measurement order to the system control device 8. The transmission request data of the measurement order includes the samples ID. The sample ID contained in the received transmission request data is stored in the process management table PT.

As shown in Fig. 6, the process management table PT stores a record related to the sample for each sample. The process management table PT includes a field F1 for storing the sample ID contained in the measurement order received from the host computer 100, a field F2 for storing the measurement item contained in the measurement order, a field F3 for storing the sample ID contained in the transmission request data of the measurement order received from the system control device 8, a field F4 for storing the measurement item related to re-test (hereinafter referred to as "retest item") when re-test is necessary, a field F5 for storing process information indicating the clinical test process which the relevant sample corresponds at the relevant time point, and a field F6 for storing error information indicating abnormality when abnormality occurs with respect to the relevant sample.

The clinical test information managing server 9 communicates with the client device 10. and provides various functions to the client device 10 in response to the request from the client device 10. For instance, when the user operates the client device 10 to instruct login to the clinical test information managing system, such login request is received and the login process is carried out. After succeeding in login, a menu screen, to be described later, is displayed on the client device 10, and thereafter, the process is executed according to the instruction from the user and the results thereof are transmitted to the client device 10. For instance, when receiving from the client device 10 a display request for a screen of a list of samples in the measurement process by the blood cell analyzer 5, information regarding the relevant sample is extracted from the process management table PT and such information regarding the sample is transmitted to the client device 10. The client device 10 receives such information, and displays a screen of a list of samples in the measurement process.

### <Configuration of client device 10>

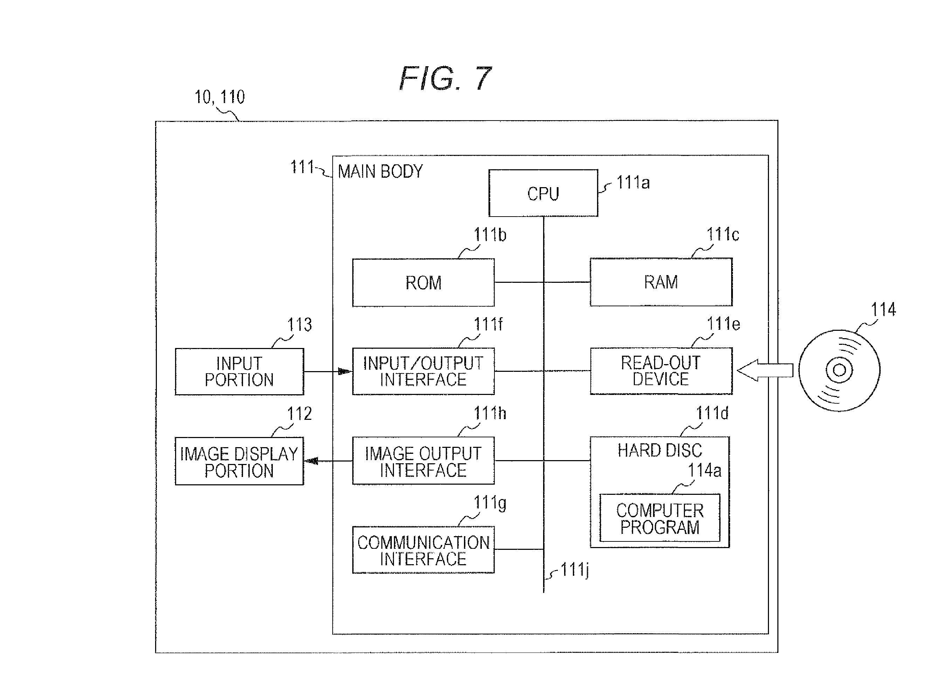

The configuration of the client device 10 will now be described. Fig. 7 is a block diagram showing a configuration of the client device 10. The client device 10 is realized by a computer 110. As shown, in Fig. 7, the computer 110 includes a main body 111, an image display portion 112, and an input portion 113. The main body 111 includes a CPU 111a, a ROM 111b, a RAM 111c, a hard disc 111d, a read-out device 111 e, an input/output interface 111 f, communication interfaces 111 g, 111h, 111i, and an image output interface 111j, where the CPU 111 a, the ROM 111b, the RAM 111c, the hard disc 111d, the read-out device 111 e, the input/output interface 111f, the communication interface 111g, the communication interface 111h, the communication interface 111 i, and the image output interface 111j are connected by a bus 111k.

The read-out device 111 e can read out a computer program 114a for causing the computer to function as the client device 10 from a portable recording medium 114. and install the computer program 114a in the hard disc 111d.

### [Operation of clinical test information managing system]

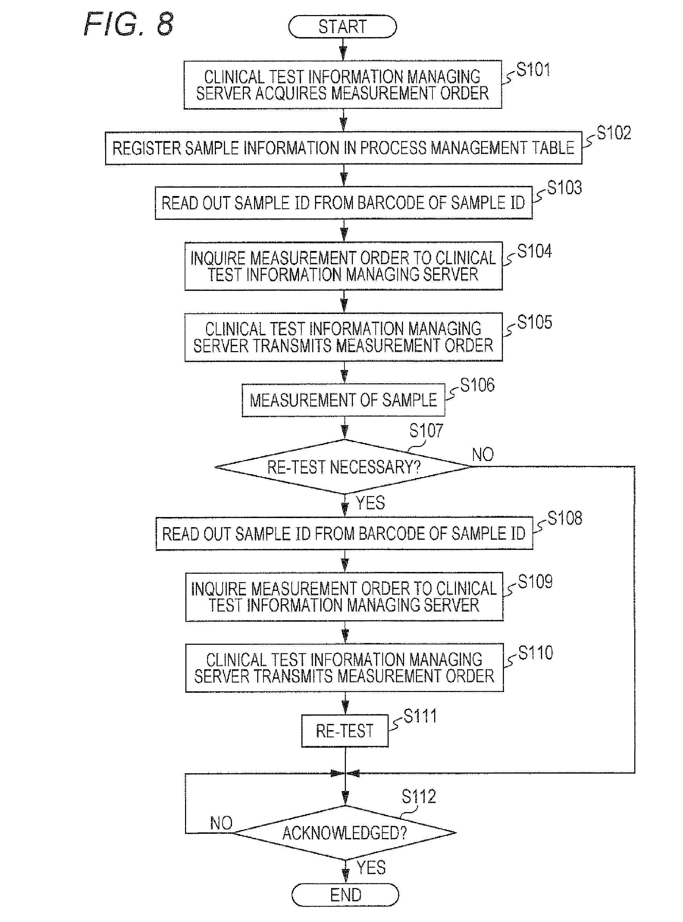

First, the flow of the entire clinical test will be described. Fig. 8 is a flowchart showing a flow of the entire clinical test. The measurement items of the clinical test necessary for the patient are determined by the doctor, and the clinical test order (measurement order) including such measurement items is registered in the host computer 100. The host computer 100 transmits the registered measurement order to the clinical test information managing server 9. The clinical test information managing server 9 receives the measurement order (step S101) and registers the data of the sample ID and the measurement item contained in the measurement order in the process management table PT as a new record (step S102). In this case, information "AP" indicating the measurement standby or the target of the measurement process is registered in the field F5 of the process management table PT as process information of the relevant record.

The blood sample collected from the patient is stored in the sample container, and a barcode label printed with a barcode indicating the sample ID contained in the measurement order is attached to the sample container. The sample container is held in the sample rack along with other sample containers, and the sample rack is mounted in the sample inserting device 2. The sample rack mounted in the sample inserting device 2 is transferred to the barcode reading unit 22, and the sample ID is read from the barcode of the sample container by the barcode reading unit 22 (step S103). The read sample ID is transmitted to the system control device 8, and the system control device 8 generates measurement order transmission request data including the sample ID, and transmits the same to the clinical test information managing server 9 (step S104). The clinical test information managing server 9 that received the sample ID from the barcode reading result matches the sample ID contained in the received transmission request data and the sample ID registered in the process management table PT, and transmits the measurement order including the measurement item corresponding to the sample ID to the system control device 8 when the sample ID that matches the sample, ID contained in the transmission request data is found from the process management table PT (step S105). In this case, the clinical test information managing server 9 changes the process information from "AP" to "P" indicating in-measurement in the record including the sample ID of the process management table PT.

When receiving the measurement order, the system control device 8 determines the transporting destination based on the measurement order and transmits instruction data to the sample transporting device 3 so as to transport the sample rack to the transporting destination. The instruction data is contained in the measurement order, and such measurement order is transmitted to the blood cell analyzer 5 of the transporting destination. The samples rack is transported to the blood cell analyzer 5 of the transporting destination by the sample transporting device 3, and the sample is measured according to the measurement order by the blood cell analyzer 5 (step S106).

After the measurement is finished, the measurement result is transmitted from the blood cell analyzer 5 to the clinical test information managing server 9. The clinical test information managing server 9 stores the measurement result in the hard disc 91d, and determines the necessity of re-test based on the measurement result. The re-test includes the re-measurement by the blood cell analyzer 5, the white blood cell automatic classification (hereinafter referred to as "automatic classification") by the blood cell image display device 7, and the test (hereinafter referred to as "microscope test") by the microscope. That is, the clinical test information managing server 9 determines whether the re-measurement by the blood cell analyzer 5 is necessary, the white blood cell automatic classification by the blood cell image display device 7 is necessary, the microscope test is necessary, or the re-test is not necessary for the sample by analyzing the measurement result of the sample (step S107). When determined that the re-measurement by the blood cell analyzer 5 is necessary, the measurement item of such re-measurement is determined.

When determined that the re-test is not necessary (NO in step S107), the process proceeds to step S 112. In this case, the clinical test information managing server 9 changes "P" of the process information to "PR" indicating wait for acknowledgement of the measurement result in the record including the sample ID of the process management table PT. When determined that the re-test is not necessary (YES in step S107), the re-test item is registered in the field F4 in the record of the corresponding sample of the process management table PT. and the process information is registered in the field F5. In this case, "AR" is registered as the process information when determined that the re-measurement by the blood cell analyzer 5 is necessary, "HG" is registered as the process information when determined that the automatic classification is necessary, and "AF" is registered as the process information when determines that the microscope test is necessary.

The sample rack for accommodating the sample, which measurement is completed, is transported by the sample transporting device 3, and accommodated in the sample accommodating device 4. The user operates the client device 10 and accesses the clinical test information managing server 9, and the like to check whether any sample, which measurement is finished, is determined that re-test is necessary. The user checks the necessity of re-test, and then again mounts the sample rack holding the sample determined that the re-test is necessary to the sample inserting device 2. Thereafter, the sample barcode is read (step S108) and the measurement order transmission request to the clinical test information managing server 9 is made (step S109), similar to steps S103 and S104 described above. The clinical test information managing server 9 that received the sample ID from the barcode reading result matches the sample ID contained in the received transmission request data and the samples ID registered in the process management table PT, and transmits the re-test order including the re-test item corresponding to the sample ID to the system control device 8 when the sample ID that matches the sample ID contained in the transmission request data is found from the process management table PT (step S110). In this case, the clinical test information managing server 9 changes the process information from "AR", "HG", or "AF" to "R", "G", or "F" indicating in-retest in the record including the sample ID of the process management table PT. "R" means during re-test by the blood cell analyzer 5, "G" means during re-test by the blood cell image display device 7, and "F" means during observation of the blood smear created by the smear creating device 6.

When receiving the re-test order, the system control device 8 determines the transporting destination based on the re-test order, and transmits the instruction data to the sample transporting device 3 so as to transport the sample rack to the transporting destination. The sample rack is transported to the instructed transporting destination, and the re-test is carried out (step S111).

After the re-test is finished, the re-test result is transmitted from the blood cell analyzer 5 or the blood cell image display 7 to the clinical test information managing server 9. The clinical test information managing server 9 stores the re-test result in the test information database in the hard disc 91d, and changes "R", "G", or "F" of the process information to "RR" indicating wait for acknowledgement of the measurement result in the record including the sample ID of the sample of the process management table PT. The user operates the client device 10 to access the clinical test information managing server 9, display the measurement result, and input the acknowledgement, thereof (step S112). When the input of acknowledgement of the re-test is received in the clinical test information managing server 9 (YES in step S112), the acknowledgement information of the re-test result is stored in the test information database in the hard disc 91 d and the process information "RR" is deleted from the record of the sample of the process management table PT. The clinical test of one sample is thereby finished.

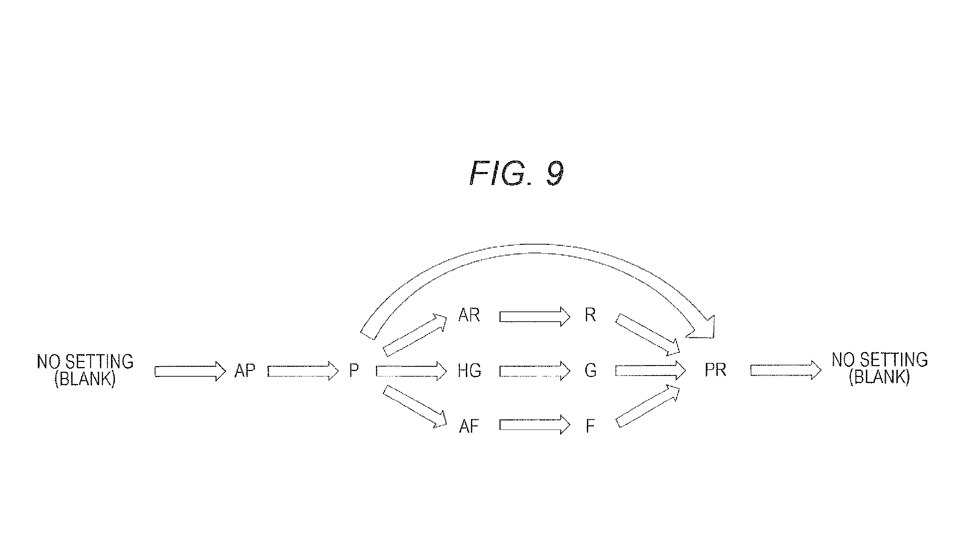

Fig. 9 is a view showing the transition of the clinical test process in the present embodiment, that is, the transition of the process information registered in F5 of the process management table. As shown in Fig. 9, the clinical test process is configured by each process of "AP" indicating measurement standby, "P" indicating measurement, "AR", "HG" or "AF" indicating re-test standby, "R", "G" or "F" indicating during re-test, and "PR" indicating acknowledgement standby. When re-test is not necessary, the process of "AR", "HG", "AF", "R", "G", and "F" are skipped.

### <Process management of clinical test>

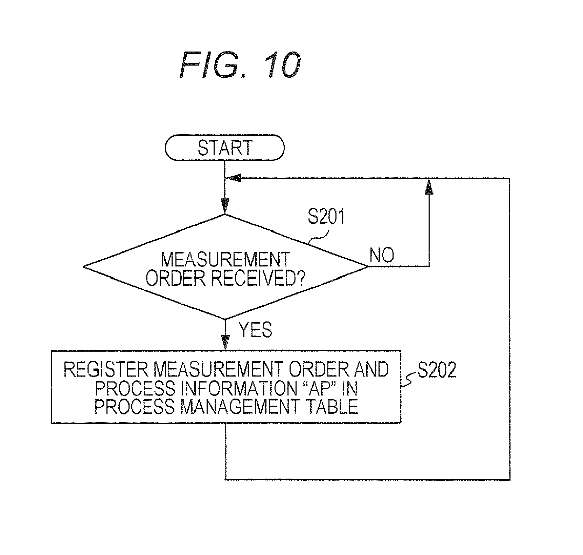

The operation related to the process management of the clinical test of the clinical test information managing server 9 will now be described. First, the measurement order registration process will be described. Fig. 10 is a flowchart showing the flow of the measurement order registration process of the clinical test information managing server 9. The CPU 91a of the clinical test information managing server 9 waits for the reception of the measurement order from the host computer 100 (NO in step S201). When the clinical test information managing server 9 receives the measurement order (YES in step S201), the CPU 91 a registers the relevant measurement order in the process management table PT (step S202). In this case, the CPU 91a registers the process information "AP" in the field F5 of the newly added record of the process management table PT. After executing the process of step S202, the CPU 91a returns the process to step S201. Thus, the measurement order registration process described above is repeatedly executed.

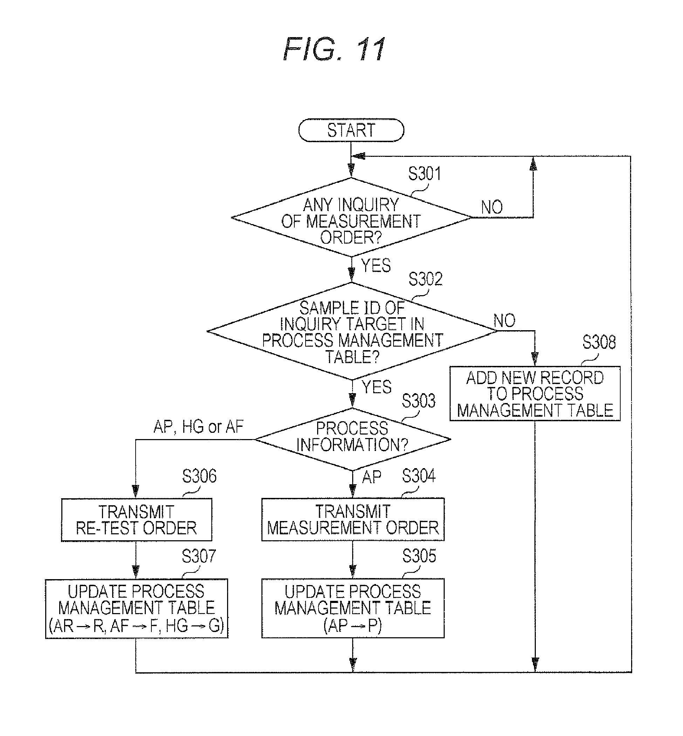

The measurement order inquiry accepting process of the clinical test information managing server 9 will now be described. Fig. 11 is a flowchart showing the flow of the measurement order inquiry accepting process of the clinical test information managing server 9. The measurement order inquiry accepting process is a process related to the update of the process management table PT when the clinical test information managing server 9 receives the measurement order transmission request data from the system control device 8. The CPU 91a of the clinical test information managing server 9 waits for the reception of the measurement order transmission request data from the system control device 8 (NO in step S301). When the clinical test information managing server 9 receives the measurement order transmission request data (YES in step S301), the CPU 91a references the process management table PT, and determines whether or not the sample ID that matches the sample ID contained in the received measurement order transmission request data is registered in the process management table PT as the sample ID contained in the measurement order (step S302).

The inquiry of the measurement order is carried out on the sample which is inserted to the sample inserting device 2 and which sample barcode is read by the barcode reading unit 22. The sample inserted to the sample inserting device 2 is not only the target of execution of the first measurement (hereinafter referred to as "initial test") but also the target of second and subsequent measurement or microscope test, that is, re-test. In the process management table PT, the process information of the sample that is the target of initial test is "AP", and the process information of the sample that is the target of re-test is "AR", "HG", or "AF". When the sample ID that matches the sample ID contained in the received measurement order transmission request data is found in the process management table PT (YES in step S302), the CPU 91a specifies the process information in the record including the sample ID (step S303).

If the specified process information is "AP" in step S303 ("AP" in step S303), the relevant sample is in the state waiting for measurement. Therefore, in this case, the CPU 91a reads out the relevant measurement order from the hard disc 91d. and transmits the measurement order to the system control device 8 (step S304). The CPU 91a also updates the process information contained in the record of the sample in the process management table PT from "AP" to "P" (step S305), and returns the process to step S301.

If the specified process information, is "AR", "HG", or "AF" in step S303 ("AR, HG or AF" in step S303), the relevant sample is in the state waiting for re-test. Therefore, in this case, the CPU 91a reads out the relevant re-test order from the hard disc 91d, and transmits the re-test order to the system control device 8 (step S306). The CPU 91a also updates the process information contained in the record of the sample, in the process management table PT to "R" if "AR", updates to "G" if "HG", and updates to "F" if "AF" (step S307). The CPU 91a returns the process to step S301 after such processes are finished.

When, in step S302, the sample ID that matches the sample ID contained in the received measurement order transmission request data is not found in the process management table PT (NO in step S302), the measurement order corresponding to such sample ID is not registered. In this case, the CPU 91 a adds a new record to the process management table PT (step S308). In this process, the CPU 91a registers the sample ID contained in the received measurement order transmission request data in the field F3 in the record, and registers the information indicating order unknown in the field F6. After such process is finished, the CPU 91 a returns the process to step S301.

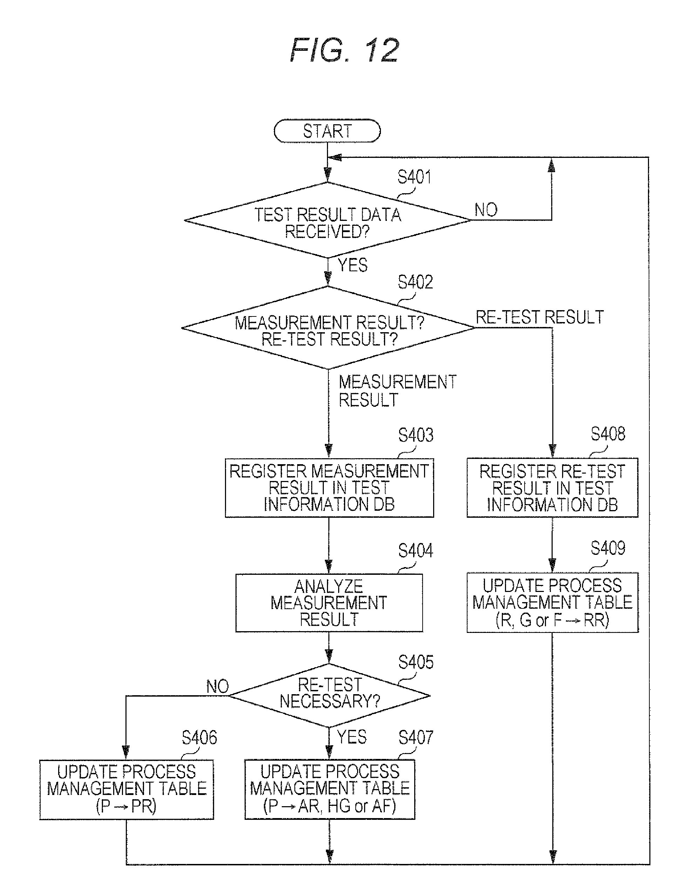

The test result accepting process of the clinical test information managing server 9 will now be described. Fig. 12 is a flowchart showing the flow of the test result accepting process of the clinical test information managing server 9. The test result accepting process is a process related to the update of the process management table PT of when the clinical test information managing server 9 receives the measurement result of the first time from the blood cell analyzer 5, or receives the re-test result from the blood cell analyzer 5 or the blood cell image display device 7.

The CPU 91a of the clinical test information managing server 9 waits for the reception of the test result data (data indicating measurement result or re-test result) (NO in step S401). When the clinical test information managing server 9 receives the test result data (YES in step S401), the CPU 91a matches the sample ID contained in the received test result data and the sample ID of each record of the process management table PT, searches for the record in which the sample ID matches, and references the process information included in the relevant record to specify whether the test result data is the measurement result of the first time or is the re-test result (step S402).

When the test result data is the measurement result of the first time in step S402 ("measurement result" in step S402), the CPU 91a registers the measurement result in the test information database of the hard disc 91d (step S403), and executes the analyzing process of the measurement result (step S404). In the analyzing process whether or not the measurement result is normal, whether or not the re-test is necessary if the measurement result is abnormal, whether or not one of re-measurement by the blood cell analyzer 5, automatic classification or microscope test is necessary if re-test is necessary are determined, where the re-test item is determined when re-measurement by the blood cell analyzer 5 is necessary.

When determined that the re-test is not necessary after the above analyzing process is executed (NO in step S405), the CPU 91a changes the process information "P" contained in the record of the sample in the process management table PT to "PR" (step S406) and returns to the process to step 5401. When determined that the re-test is necessary (YES in step S405), the CPU 91a registers the re-test item in the record of the sample in the process management table PT, changes the process information of the record from "P" to "AR" when the re-measurement by the blood cell analyzer 5 is necessary, changes the process information of the record from "P" to "HG when the automatic classification is necessary, and changes the process information of the record from "P" to "AF" when the microscope test is necessary (Step S407). The CPU 91a returns the process to step S401 after such processes are finished.

When the test result data is the re-test result in step S402 ("re-test result in step S402), the CPU 91 a registers the relevant re-test result in the test information database of the hard disc 91d (step S408), and changes the process information "R", "G", or "F" contained in the record of the sample in the process management table PT to "RR" (step S409). The CPU 91a returns the process to step S401 after such processes are finished.

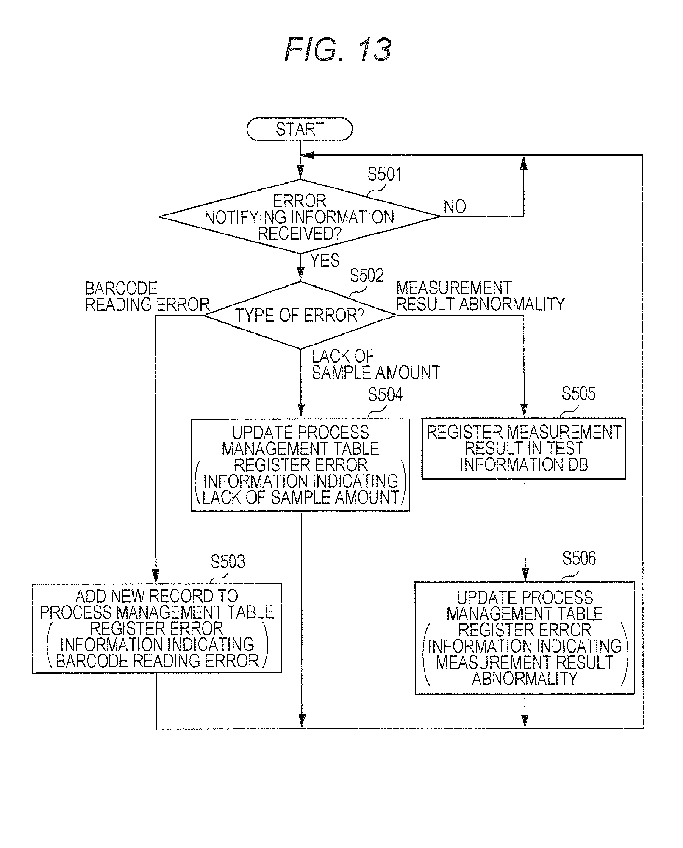

The error notifying information accepting process of the clinical test information managing server 9 will now be described. Fig. 13 is a flowchart showing the flow of the error notifying information accepting process of the clinical test information managing server 9. The error notifying information accepting process is a process related to the update of the process management table PT when the clinical test information managing server 9 accepts the error notifying information.

When an error related to the sample or an error related to the device occurs, the sample inserting device 2, the system control device 8, the blood cell analyzer 5, the smear creating device 6, and the blood cell image display device 7 transmit error notifying information to the clinical test information managing server 9. The error notifying information includes an error code corresponding to the type of error, so that the type of error can be specified. If the error is related to the sample, the error notifying information includes the sample ID of the sample. For instance, when a reading error of the barcode occurs in the barcode reading unit 22. the error notifying information including the error code of barcode reading error is transmitted. When the sample amount is less than the defined amount, and measurement or smear creation cannot be carried out, the error notifying information including an error code indicating lack of sample amount and the sample ID of the sample is transmitted. Furthermore, when abnormality is found in the numerical value of the measurement result, the fractionation in the scattergram and the like, the error notifying information including the measurement result, the error code indicating measurement result abnormality, and the sample ID of the sample is transmitted.

The CPU 91a of the clinical test information managing server 9 waits for the reception of the error notifying information (NO in step S501). When the clinical test information managing server 9 receives the error notifying information (YES in step S501), the CPU 91a analyzes the received error notifying information and distinguishes the type of error related to the relevant error notifying information (step S502).

When the type of error is the barcode reading error ("barcode reading error" in step S502), the CPU 91a registers a new record related to the error notifying information in the process management table PT (step S503). In this process, the CPU 91 a generates an error sample ID unique to the error notifying information in place of the sample ID related to the barcode reading error, and registers the error sample ID in the field F3 of the new record and the error information indicating the barcode reading error in the field F6. In this case, the CPU 91a deletes the process information from the field F5 of the record. The CPU 91a returns the process to step S 50 after such process is finished.

When the type of error is lack of sample amount ("lack of sample amount" in step S502), the CPU 91a updates the record in which the sample ID included in the error notifying information is registered in the process management table PT (step S504). In this process, the CPU 91a searches for the record in which the sample ID same as the sample ID included in the error notifying information is registered in the field F1 in the process management table PT, and registers the error information of lack of sample amount in the field F6 of the record. In this case, the CPU 91a deletes the process information from the field F5 of the record. The CPU 91a returns the process to step S501 after such process is finished.

When the type of error is measurement result abnormality ("measurement result abnormality" in step S502), the CPU 91a registers the measurement result included in the error notifying information in the test information database (step S505), and updates the record in which the sample ID included in the error notifying information is registered in the process management table PT (step S506). In the process of step S506, the CPU 91a searches for the record in which the sample ID same as the sample ID included in the error notifying information is registered in the field F1 in the process management table PT, and registers the error information of measurement result abnormality in the field F6 of the record. In this case, the CPU 91a deletes the process information from the field F5 of the record. The CPU 91a returns the process to step S501 after such process is finished.

Various types of errors exist other than the above errors. Although omitted here, in the error notifying information accepting process, the CPU 91a detects the type of error when accepting such error notifying information, and executes the process corresponding to the type of error.

Other than the above, a process in which the sample information, where measurement is executed but the transmission request of the measurement order is not made, is specified from the process management table PT, the error information indicating "no ID" is registered in the field F6 of the record, and the process information is deleted from the field F5.

### <Display of clinical test information management screen>

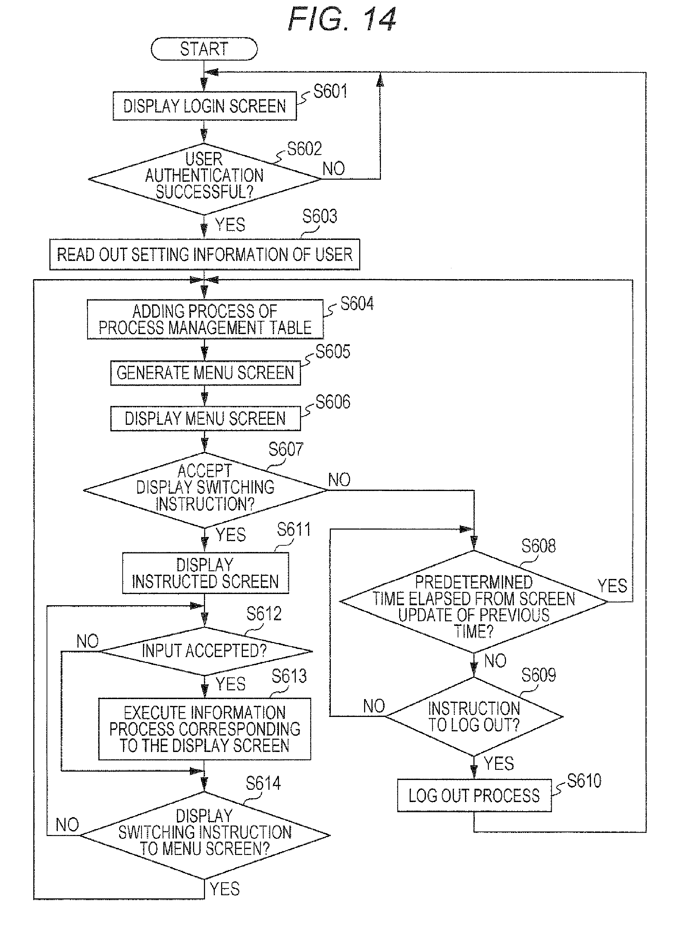

The operation related to the display of the clinical test information managing server 9 will now be described. Fig. 14 is a flowchart showing the flow of the display process of the clinical test information management screen of the clinical test information managing server 9. When browsing through or operating the information, of the sample related to clinical test using the clinical test information managing server 9, the user needs to log into the clinical test information managing server 9. The CPU 91a first displays a login screen for the user to log into the clinical test information managing system on the image display unit 92 (step S601). The login screen (not shown) includes an input box for inputting the user name and the password, an OK button for confirming the input user name and the password, and a cancel button for canceling the input user name and the password. The user performs the operation of inputting the user name and the password corresponding thereto, and then selecting the OK button. The CPU 91a executes the user authentication process using the user name and the password, which inputs are received in the above manner (step S602).

When the user name and the password do not match those that are registered and the authentication of the user fails in the user authentication process (NO in step S602), the CPU 91a returns the process to step S601 and again displays the login screen. When the user name and the password match those that are registered and the authentication of the user is successful in the user authentication process (YES in step S602), the CPU 91a a reads out the setting information of the user from the hard disc 91d (step S603). Thereafter, the CPU 91a adds up the number of samples (number of records) for every process information and every error information with reference to the process management table PT (step S604), generates a menu screen based on the setting information read in step S603 and the added result of step S604 (step S605), and displays the menu screen S605 on the image display unit 92 (step S606).

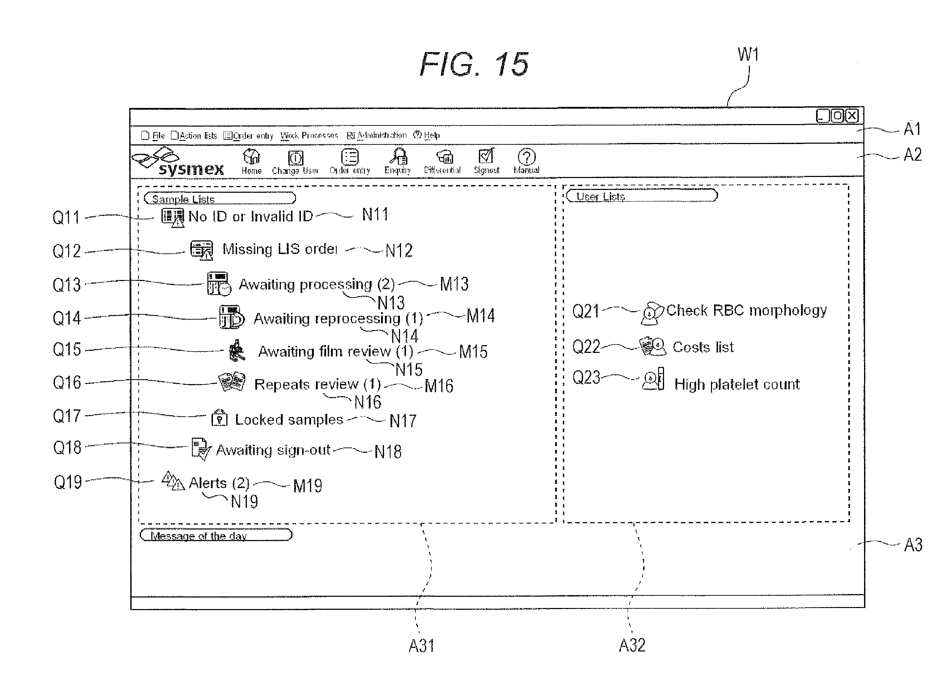

Fig. 15 is a view showing one example of a menu screen. As shown in the figure, the menu screen W1 includes a menu bar region A1 at the upper part of the screen, a tool bar region A2 at the lower side thereof, and a work region A3 arranged on the lower side of the tool bar region A2 and occupying most part of the menu screen W1. The regions A1 to A3 are commonly arranged in all the display screens of the clinical test information managing server 9. In the menu bar region A1, menus such as "File", "Enquiry (information query)", and "Administration (management)" are lined, where each menu can be selected by operating the mouse to display the pull-down menu. A plurality of menus are lined in each pull-down menu, where one of the menus is selected by operating the mouse to execute the relevant menu. A plurality of icons is lined in the tool bar region A2. The names of icons such as "Home" and "Enquiry (information query)" are displayed on the lower side of each icon.

The work region A3 includes a fixed icon region A31 on the left side and a variable icon region A32 on the right side as shown in Fig. 15. The fixed icon region A31 includes a plurality of icons Q11 to Q19. In the fixed icon region A31, the icons to be displayed cannot be determined by the setting of the user and the icons Q11 to Q19 defined in advance are fixedly displayed.

The name (character string) N11 of "No ID or Invalid ID" is displayed next to the icon Q11, and the function for switching the display to the ID unknown sample list screen that displays in a list the information regarding the sample with unknown ID is assigned to the icon Q11. If the information regarding the sample displayed in the ID unknown sample list screen exists, the sample number information indicating the number of such samples is displayed next to the name N11.

The name (character string) N12 of "Missing LIS order " is displayed next to the icon Q12, and the function for switching the display to the measurement order unknown sample list screen that displays in a list the information regarding the sample with unknown measurement order is assigned to the icon Q12. If the information regarding the sample displayed in the measurement order unknown sample list screen exists, the sample number information indicating the number of such samples is displayed next to the name N12.

The name (character string) N13 of "Awaiting processing (waiting for measurement) " is displayed next to the icon Q13, and the function for switching the display to the measurement awaiting sample list screen that displays in a list the information regarding the sample which measurement order is acquired but is not yet subjected to measurement is assigned to the icon Q13. If the information regarding the sample displayed in the measurement awaiting sample list screen exists, the sample number information M13 indicating the number of such samples is displayed next to the name N13.

The name (character string) N14 of "Awaiting reprocessing (waiting for re-measurement)" is displayed next to the icon Q14, and the function for switching the display to the remeasurement awaiting sample list screen that displays in a list the information regarding the sample which re-test order is acquired but is not yet subjected to re-measurement is assigned to the icon Q14. If the information regarding the sample displayed in the remeasurement awaiting samples list screen exists, the sample number information M 14 indicating the number of such samples is displayed next to the name N14.

The name (character string) N15 of "Awaiting film review (waiting for microscope test)" is displayed next to the icon Q15, and the function for switching the display to the awaiting film review sample list screen that displays in a list the information regarding the sample which re-test order is acquired but is not yet subjected to microscope test is assigned to the icon Q15. If the information regarding the sample displayed in the awaiting film review sample list screen exists, the sample number information M15 indicating the number of such samples is displayed next to the name N15.

The name (character string) N16 of "Repeats review (re-test review)" is displayed next to the icon Q16, and the function for switching the display to the acknowledgement awaiting sample list screen that displays in a list the information regarding the sample in which acknowledgment of the measurement result is not carried out is assigned to the icon Q16. If the information regarding the sample displayed in the acknowledgement awaiting sample list screen exists, the sample number information M16 indicating the number of such samples is displayed next to the name N16.

The name (character string) N17 of "Locked samples " is displayed next to the icon Q17, and the function for switching the display to the locked sample list screen that displays in a list the information regarding the sample locked (operation disabling change, edit, and delete) so that the user (super user) having management authority can again consider the measurement result is assigned to the icon Q17. If the information regarding the sample displayed in the locked sample list screen exists, the sample number information indicating the number of such samples is displayed next to the name N17.

The name (character string) N18 of "Awaiting sign-out " is displayed next to the icon Q18, and the function for switching the display to the awaiting sign-out sample list screen that displays in a list the information regarding the sample awaiting for a predetermined sing-out is assigned to the icon Q18. If the information regarding the sample displayed in the awaiting sing-out sample list screen exists, the sample number information indicating the number of such samples is displayed next to the name N18.

The name (character string) N19 of "Alerts " is displayed next to the icon Q19, and the function for switching the display to the alert sample list screen that displays in a list the information regarding the sample that has been issued an alert with the detection of abnormality is assigned to the icon Q19. If the information regarding the sample displayed in the alert sample list screen exists, the sample number information M19 indicating the number of such samples is displayed next to the name N19.

The variable icon region A32 includes a plurality of icons Q21 to Q23. In the variable icon region A31, the icons to be displayed can be determined by the setting by the user, and the icons Q21 to Q23 display-set by the user are displayed. The icons to be displayed in the variable icon region A32 can be changed by the setting by the user.

The "icon" referred to here is an example of an object operated by the user when changing the displayed screen to a predetermined screen defined in advance, and includes an image assigned with a specific function, designed to symbolically represent such function and displayed within the window.

The sample number information displayed next to the names N11 to N19 of the icons displayed in the fixed icon region A31 will now be described in detail below, In step S604, the CPU 91a adds up the number of records having the same process information in the process management table PT for every process information. For instance, the number of records in which the process information is "AP", the number of records in which the process information is "AR", and the number of records in which the process information is "AF" are counted respectively. In the process of step S604, the CPU 91a counts the number of records registered with the error information in the process management table PT, and adds up the number of records having the same error information for every error information. For instance, the number of records in which the error information is "order unknown", the number of records in which the error information is "barcode reading error", and the number of records in which the error information is "no ID" are counted respectively.

After adding up as above, the CPU 91a generates a menu screen in which the corresponding process information or the sample number information, which is the result of adding up for the error information, is displayed next the name of each icon in step S605. More specifically describing, the number of records in which the error information is "no ID" is displayed next to the name N11, and the number of records in which the error information is "order unknown" is displayed next to the name N12 as sample number information. More specifically describing, the number of records in which the process information is "AP" is displayed next to the name N13, the number of records in which the process information is "AR" is displayed next to the name N14, the number of records in which the process information is "AF" is displayed next to the name N15, the number of records in which the process information is "RR" is displayed next to the name N16, and the total number of records registered with the error information is displayed next to the name N19 as sample number information.

The user can select the icon displayed in the work region A3 by performing the operation of clicking the left button of the mouse with such menu screen displayed, where when the icon is selected, the screen corresponding to the icon is displayed. That is, for example, when the icon Q11 is selected, the display switches to the ID unknown sample list screen, and when the icon Q12 is selected, the display switches to the measurement order unknown sample list screen. The CPU 91a determines whether or not the selection of the icon, that is, the screen display switching instruction from the user is accepted (step S607).

If the screen display switching instruction is not accepted in step S607 (NO in step S607), the CPU 91 a determines whether or not a predetermined time (e.g., 20 seconds) has elapsed from the update of the menu screen of the previous time (if menu screen is not updated before that, whether or not a predetermined time has elapsed from when the menu screen is displayed) (step S608). If the predetermined time has not elapsed (NO in step S608), the CPU 91 a determines whether or not the instruction to log out is accepted (step S609). If the instruction to log out is accepted (YES in step S609), the CPU 91a executes the log out process (step S610), and returns the process to step S601.

If the instruction to log out is not accepted in step S609 (NO in step S609), the CPU 91 a returns the process to step S608, and repeats the processes of steps S608 and S609 until the elapse of a predetermined time. If a predetermined time has elapsed in step S609 (YES in step S609), the CPU 91a returns the process to step S604, again adds up the number of samples for every process information and every error information, re-generates the menu screen, and updates the display of the menu screen.

If the screen display switching instruction is accepted in step S607 (YES in step S607), the CPU 91a switches the display to the screen corresponding to the selected icon (step S611). The CPU 91a determines whether or not the instruction for information processing corresponding to the display screen is accepted from the user (step S612), and proceeds the process to step S614 if the instruction is not accepted. If the instruction for information processing corresponding to the display screen is accepted (YES in step S612), the CPU 91a executes the information processing corresponding to the display screen (step S613), and proceeds the process to step 5614.

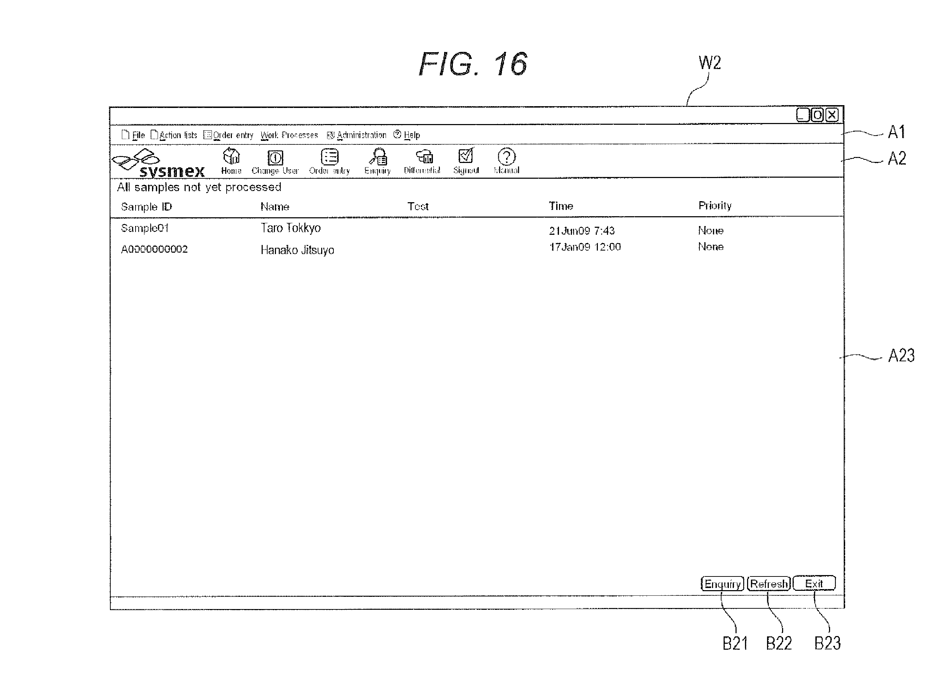

The processes of steps S613 and S614 will be specifically described. For instance, when the icon Q13 is selected in the menu screen W1, the measurement awaiting sample list screen is displayed. Fig. 16 is a view showing one example of a measurement awaiting sample list screen. As shown in the figure, the measurement awaiting sample list screen W2 also includes the menu bar region A1, the tool bar region A2, and the work region A23, similar to the menu screen W1. The menu included in the menu bar region A1 of the measurement awaiting sample list screen W2 is the same as the menu of the menu bar region A1 of the menu screen W1. The icons in the tool bar region A2 of the measurement awaiting sample list screen W2 are the same as the icons of the tool bar region A2 of the menu screen W1. The menu and the icons same as the menu screen W1 are provided to instruct the executable processes in the menu screen W1, and such processes can be given instruction to be executed in the measurement awaiting sample list screen.

The information regarding one or a plurality of samples can be displayed in a list in the form of a table in the work region A23 of the measurement awaiting sample list screen W2. The sample information is displayed by one sample in each row in the work region A23. The work region A23 includes a plurality of columns, where the information such as the sample ID, the name of the patient, the time the clinical test information managing server received the measurement order, and the priority of the measurement are respectively displayed in such columns. The sample information displayed in such manner can be selected through the operation of clicking the left button of the mouse for every row. A plurality of sample information may be selected all at once. Three buttons, the "Enquiry (information query)" button B21, the "Refresh (screen update)" button B22, and the "Exit (display end)" button B23 are displayed near the lower end of the work region A23. The button B21 is a button for displaying the detailed information of the sample, where more detailed information of the selected sample information is displayed when the button B21 is selected through the operation of clicking the left button of the mouse with the sample information selected. The button B22 is a button for updating the screen, where the screen is updated by the most recent sample information when the button B22 is selected. The button B23 is a button for terminating the display of the measurement awaiting sample list screen W2, where the display switching instruction to the menu screen W1 is given to the CPU 91a and the display is switched to the menu screen W1 when the button B23 is selected.

The sample information displayed in the relevant measurement awaiting sample list screen W2 is the sample information regarding the record having the process information "AP" in the process management table PT. That is, the sample information for the sample having the process information "AP" in the process management table PT is displayed in a list in the measurement awaiting sample list screen W2. The number of sample information displayed in the measurement awaiting sample list screen W2 is the same as the number of sample information M13 displayed in correspondence with the icon Q13 next to the name N13 in the menu screen W1. In other words, the number of sample information M13 displayed in correspondence with the icon Q13 indicates the number of sample information displayed in the measurement awaiting sample list screen W2 displayed when the icon Q13 is selected, so that the user can know whether or not the sample (sample to be the target of the measurement process) waiting for the measurement process displayed in the measurement awaiting sample list screen W2 exists, and how many samples exists by simply looking at the menu screen W1 without displaying the measurement awaiting sample list screen W2.

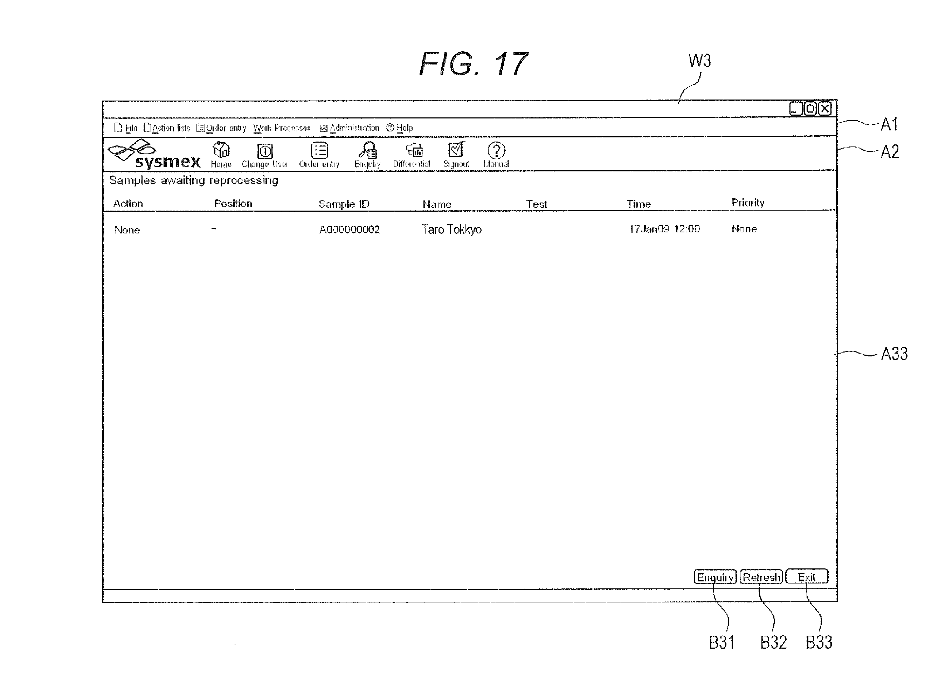

When the icon Q14 is selected in the menu screen W1, the re-measurement awaiting sample list screen is displayed. Fig. 17 is a view showing one example of a remeasurement awaiting sample list screen. Similar to the measurement awaiting sample list screen W2 described above, the remeasurement awaiting sample list screen W3 also includes the menu bar region A1, the tool bar region A2, and the work region A33.

The information regarding one or a plurality of samples can be displayed in a list in the form of a table in the work region A33 of the remeasurement awaiting sample list screen W3. The sample information is displayed by one sample in each row in the work region A33. The work region A33 includes a plurality of columns, where information on "Action" indicating what kind of re-test is being requested, "Position" indicating the sample rack and the holding position where the relevant sample is being held, "Sample ID", "Name (name of patient)", "Time" indicating the time the clinical test information managing server received the measurement order, and "Priority" indicating the priority of the measurement are respectively displayed in such columns. The sample information displayed in such manner can be selected through the operation of clicking the left button of the mouse for every row. A plurality of sample information may be selected all at once. Three buttons, the "Enquiry (information query)" button B31, the "Refresh (screen update)" button B32, and the "Exit (display end)" button B33 are displayed near the lower end of the work region A33. The button B31 is a button for displaying the detailed information of the sample, where more detailed information of the selected samples information is displayed when the button B31 is selected with the sample information selected. The button B32 is a button for updating the screen, where the screen is updated by the most recent sample information when the button B32 is selected. The button B33 is a button for terminating the display of the remeasurement awaiting sample list screen W3, where the display switching instruction to the menu screen W1 is given to the CPU 91a and the display is switched to the menu screen W1 when the button B33 is selected.

The sample information displayed in the relevant remeasurement awaiting sample list screen W3 is the sample information regarding the record having the process information "AR" in the process management table PT. That is, the sample information for the sample having the process information "AR" in the process management table PT is displayed in a list in the remeasurement awaiting sample list screen W3. The number of sample information displayed in the remeasurement awaiting sample list screen W3 is the same as the number of sample information M14 displayed in correspondence with the icon Q14 next to the name N14 in the menu screen W1. In other words, the number of sample information M14 displayed in correspondence with the icon Q14 indicates the number of sample information displayed in the remeasurement awaiting sample list screen W3 displayed when the icon Q14 is selected, so that the user can know whether or not the sample (sample to be the target of the remeasurement process) waiting for the remeasurement process displayed in the remeasurement awaiting sample list screen W3 exists, and how many samples exists by simply looking at the menu screen W1 without displaying the remeasurement awaiting sample list screen W3.

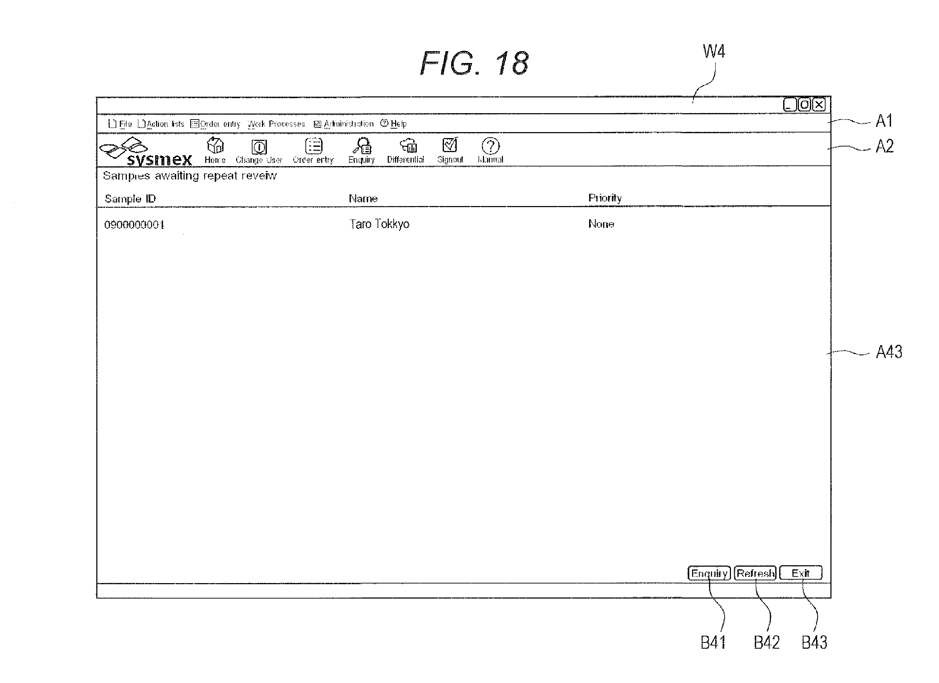

When the icon Q16 is selected in the menu screen W1, the acknowledgement awaiting sample list screen is displayed. Fig. 18 is a view showing one example of an acknowledgement awaiting sample list screen. Similar to the measurement awaiting sample list screen W2 described above, the acknowledgement awaiting sample list screen W4 also includes the menu bar region A1, the tool bar region A2, and the work region A43.

The information regarding one or a plurality of samples can be displayed in a list in the form of a table in the work region A43 of the acknowledgement awaiting sample list screen W4. The sample information is displayed by one sample in each row in the work region A43. The work region A43 includes a plurality of columns, where information on "Sample ID", "Name (name of patient)", "Time", and "Priority" are respectively displayed in such columns. The sample information displayed in such manner can be selected through the operation of clicking the left button of the mouse for every row. A plurality of sample information may be selected all at once. Three buttons, the "Enquiry (information query)" button B41 the "Refresh (screen update)" button B42, and the "Exit (display end)" button B43 are displayed near the lower end of the work region A43. The functions of the buttons B41 to B43 are similar to the functions of the buttons B21 to B23, and thus the description will be omitted.

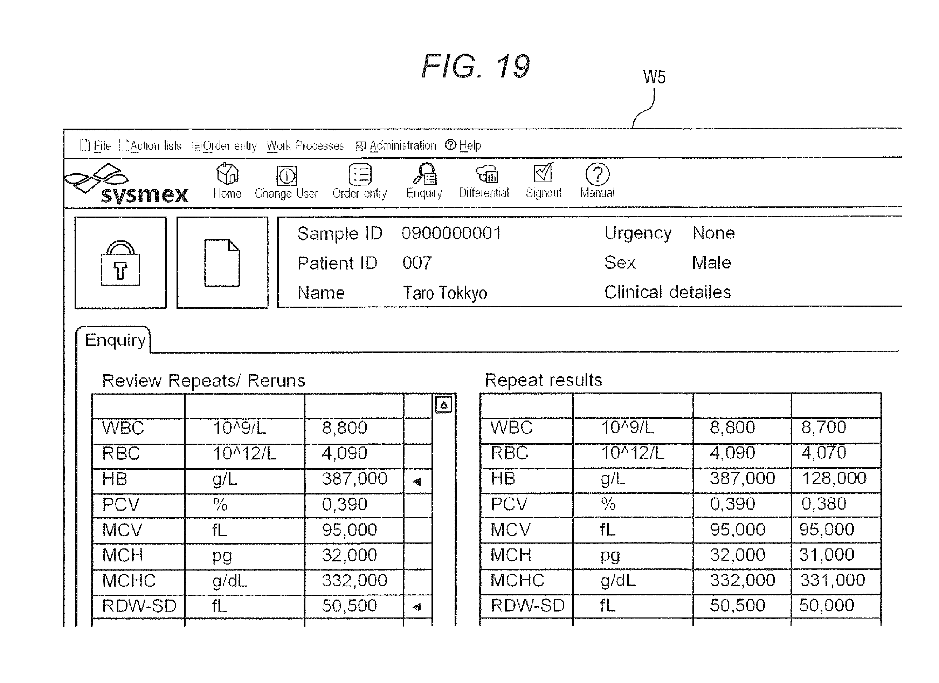

When the button B41 is selected with the sample information selected, the detailed information screen including the more detailed information of the selected sample information is displayed. Fig. 19 is a view showing one part of a detailed information screen. The detailed information screen W5 displays the sample ID, the patient number, the name of the patient, the degree of urgency, the sex, the detailed test result, and the re-test result. The detailed information screen W5 includes an acknowledgement button (not shown), where the user selects the acknowledgement button after checking the detailed information of the sample including the re-test result to acknowledge the re-test result. After the re-test result is acknowledged, the record related to the sample information of the process management table PT is updated by the CPU 91 a, and the process information "PR" contained in the record is deleted.

The sample information displayed in the acknowledgement awaiting sample list screen W4 is the sample information regarding the record including the process information "RR" in the process management table PT. That is, the sample information for the sample having the process information "RR" in the process management table PT is displayed in a list in the acknowledgement awaiting sample list screen W4. The number of sample information displayed in the acknowledgement awaiting sample list screen W4 is the same as the number of sample information M16 displayed in correspondence with the icon Q16 next to the name N 16 in the menu screen W1. In other words, the number of sample information M16 displayed in correspondence with the icon Q16 indicates the number of sample information displayed in the acknowledgement awaiting sample list screen W4 displayed when the icon Q16 is selected, so that the user can know whether or not the sample (sample to be the target of the acknowledgement process) waiting for the acknowledgement process displayed in the acknowledgement awaiting sample list screen W4 exists, and how many samples exists by simply looking at the menu screen W1 without displaying the acknowledgement awaiting sample list screen W4.

In step S614, the CPU 91a determines whether or not the display switching instruction to the menu screen (selection of the above described button B23, B33, B43) is made (step S614), and returns the process to step S612 when the display switching instruction of the menu screen is not made (NO in step S614). When the display switching instruction of the menu screen is accepted in step S614 (YES in step S614), the CPU returns the process to step S604, again executes the adding process of the process management table PT, and displays the menu screen on the image display unit 92.

As described in detail above, in the clinical test system 1 according to the present embodiment, the display unit 92 is configured to display cascading tiers of screens, and the number of samples to be displayed in the screen that is called out by the relevant icon is displayed next to the icons Q11 to Q19, and hence the presence of the sample information to be checked in each screen, and the number of samples if the sample to be checked exists can be easily checked in the menu screen W1 without displaying each screen. In particular, the icons Q13 to Q16 are assigned functions to call out the screen in which the information related to a sample in a specific process (measurement process, re-measurement process, microscope test process, acknowledgement process) of the clinical test is displayed, and the number of samples to be the target of each process is displayed in correspondence with the icons Q13 to Q16. Therefore, the user can easily grasp the presence of the sample in each process and the number of samples if the target sample exists by simply checking the menu screen.

In the present embodiment, only a case of displaying the menu screen in the clinical test information managing server 9 has been described, but the menu screen and other clinical test screens can be displayed in the client device 10. ln this case, when the user accesses the clinical test information managing server 9 using the client device 10, the log in screen is displayed in the client device 10. When the user inputs the user name and the password to the client device 10 and gives the instruction to log in, the login request is transmitted to the clinical test information managing server 9 and the login authentication is carried out, where the setting information by the user is read out if the log in is successful, and the adding process of the fixed management table PT is carried out. The setting information and the adding process result obtained in such manner are then transmitted from the clinical test information managing server 9 to the client device 10, and the client device 10 generates the menu screen based on such information and displays the menu screen. When the icons Q11 to Q19 are selected, the request data corresponding to the selected icon is transmitted to the clinical test information managing server 9, and the data necessary for the display of the screen called out by the selected icon is transmitted from the clinical test information managing server 9 to the client device 10. The client device 10 displays the screen based on the data. Instead of displaying the clinical test screen by the computer program 114a or a dedicated program for functioning the computer as the client device 10 of the clinical test information managing system, the clinical test screen may be displayed by the WWW browser executed on the client device 10. In this case, the clinical test information managing server 9 has the function of the WWW server, generates the HTML data for the menu screen, the measurement awaiting sample list screen, the re-measurement awaiting sample list screen, the microscope test awaiting sample list screen, and the acknowledgement awaiting sample list screen according to the request from the client device 10, and transmits the same to the client device 10, so that the CPU 91 a of the clinical test information managing device 9 controls the client device 10 so as to display the clinical test screen on the image display unit 112 of the client device 10.

### (Other embodiments)

In the embodiment described above, the configuration in which the icons Q11 to Q19 are arranged in the menu screen W1, and the display transitions to the screen corresponding to the selected icon when the icon Q11 to Q19 is selected has been described, but this is not the sole case. Objects other than icons such as a button or a link (character string) may be displayed on the menu screen, and the display may transition to a screen by selecting the button or the link.

If the sample, which information regarding the sample is to be displayed in the screen corresponding to the icon Q11 to Q19, exists, the number of samples is displayed in correspondence to the icon Q11 to Q19, but this is not the sole case. If the sample, which information regarding the sample is to be displayed in the screen corresponding to the icon Q11 to Q19, exists, a symbol (e.g., "*", "$". "#" etc.) may be displayed instead of numbers in correspondence to the icon, or the display of the icons Q11 to Q19 may be in a mode (color, pattern, size, etc.) different from when the sample does not exist, or the names N11 to N19 displayed next to the icons Q11 to Q19 may be displayed in a character style (thick, italic, font etc.) different from when the sample does not exist.

In the embodiment described above, a configuration of displaying the number of records in which the process information is "AP", the number of records in which the process information is "AR", the number of records in which the process information is "AF", and the like in the menu screen W1 has been described, but this is not the sole case, and the number of samples in other process such as the number of records in which the process information is "P" and the number of records in which the process information is "PR" may be displayed in the menu screen W1.

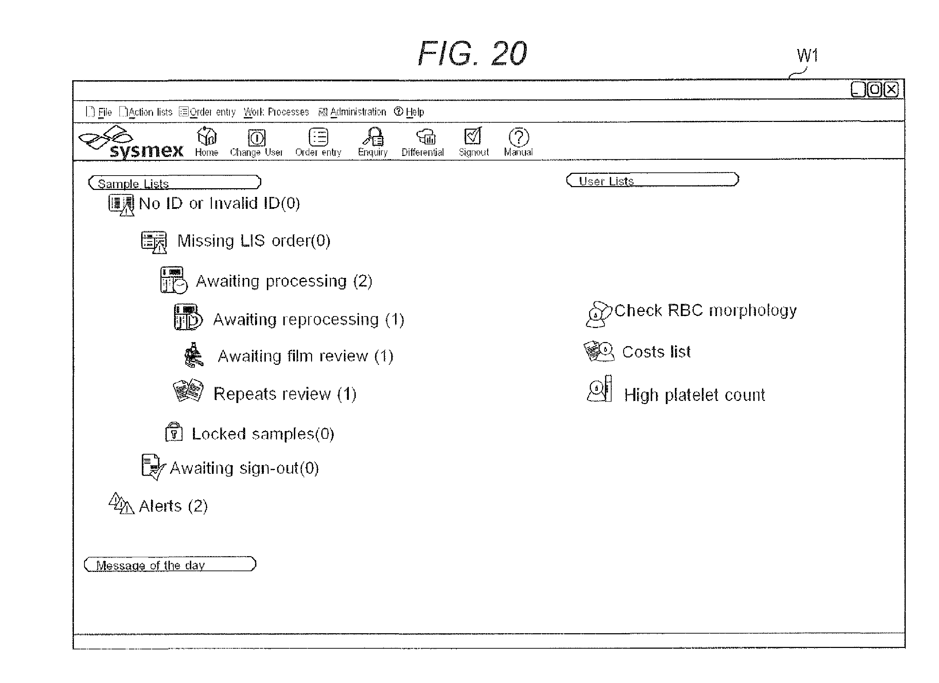

In the embodiment described above, it is shown that the relevant sample is not present by not displaying anything for the process in which the number of records is "0", but "0" may be displayed along with each name (character string) for the process in which the number of records is "0". The menu screen W1 in this case is shown in Fig. 20.

In the embodiment described above, an example in which the clinical test information managing server 9 is connected to the blood analyzing system 200, the blood cell counting device 300, the blood coagulation measurement device 400, and the urine analyzer 500 has been described, but may be connected to other clinical sample analyzers, or may be connected to only one clinical sample analyzer such as the blood analyzing system 200.

## Claims

1. A clinical test information managing apparatus comprising at least one display configured to display cascading tiers of clinical test information, the apparatus further comprising at least one processor of a computer system and at least one memory that stores programs executable by the at least one processor to:
display a first tier of clinical test information comprising a listing of items which comprise process stages that show progresses of testing, associated with an indication of presence of samples which are situated at a respective stage; and
when receiving a selection by a user among the items, display a second tier of clinical test information comprising a listing of identifications of samples which are identified by the indication of presence associated with a selected item.

2. The apparatus according to claim 1, wherein the indication of presence is a number of samples situated at the respective stage.

3. The apparatus according to claim 1 or 2, wherein the items in the first tier of clinical test information further comprise a type of an error, associated with an indication of presence of samples which sustain the error.

4. The apparatus according to any one of claims 1 to 3, wherein the second tier of clinical test information further comprises names of patients from whom the samples come.

5. The apparatus according to any one of claims 1 to 4, wherein when receiving a selection by a user among the identifications of the samples in the second tier of clinical test information, the at least one processor displays a third tier of clinical test information comprising patient information of a selected sample and any test results of the selected sample.

6. The apparatus according to any one of claims 1 to 5, wherein the at least one processor displays the items of the first tier of clinical test information by way of icons representing the items.

7. The apparatus according to any one of claims 1 to 6, wherein the at least one processor updates the clinical test information either automatically on a regular basis or upon receiving an instruction by a user.

8. The apparatus according to claim 3, wherein the type of error indicates that a sample has no identification or an invalid identification.

9. The apparatus according to claim 3, wherein the type of error indicates that a test order is missing for a sample.

10. The apparatus according to any one of claims 1 to 9, wherein the process stages comprise:
a stage that indicates that a sample is waiting for initial testing;
a stage that indicates that initial testing for a sample is in progress; and
a stage that indicates that a test results for a sample is waiting for acknowledgement.

11. The apparatus according to any one of claims 1 to 10, wherein the process stages comprise:
a stage that indicates that a sample is waiting for re-testing;
a stage that indicates that re-testing is in progress on a sample;

12. The apparatus according to claim 10, wherein when receiving a selection of the stage in the first tier of clinical test information which indicates that a sample is waiting for initial testing, the at least one processor displays the second tier of clinical test information which comprises a time at which a test order is received for a respective sample.

13. The apparatus according to claim 11, wherein when receiving a selection of the stage in the first tier of clinical test information which indicates that a sample is waiting for re-testing, the at least one processor displays the second tier of clinical test information which comprises a type of re-test to be performed on a sample and a position of a sample if the sample is with other samples in a rack.

14. The apparatus according to claim 10, wherein
when receiving a selection of the stage in the first tier of clinical test information which indicates that a test results for a sample is waiting for acknowledgement, the at least one processor displays the second tier of clinical information which comprises a listing of identifications of samples which are situated in the selected stage, and
when further receiving a selection of an sample identification in the second tier of clinical test information, the at least one processor displays the third tier of clinical test information which comprises test results of the sample, which may be acknowledged by a user on display.

15. A non-transitory storage medium which stores computer-executable programs executed by at least one processor of a computer system to:
display a first tier of clinical test information comprising a listing of items which comprise process stages that show progresses of testing, associated with an indication of presence of samples which are situated at a respective stage; and
when receiving a selection by a user among the items, display a second tier of clinical test information comprising a listing of identifications of samples which are identified by the indication of presence associated with a selected item.
